(19) Europäisches Patentamt
European Patent Office
Office européen des brevets

(11) Veröffentlichungsnummer: **0 385 264 A2**

(12) # EUROPÄISCHE PATENTANMELDUNG

(21) Anmeldenummer: **90103411.6**

(22) Anmeldetag: **22.02.90**

(51) Int. Cl.5: **C07C 33/025, C07C 33/02, C07C 49/24, C12P 7/18, C12P 7/26, C12N 1/20, C12N 9/99, //(C12N1/20, C12R1:465)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4356.

(30) Priorität: **25.02.89 DE 3905930**

(43) Veröffentlichungstag der Anmeldung: **05.09.90 Patentblatt 90/36**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Zeeck, Axel, Prof. Dr.**
**Brüder-Grimm-Allee 22**
**D-3400 Göttingen(DE)**
Erfinder: **Grabley, Susanne, Dr.**
**Hölderlinstrasse 7**
**D-6240 Königstein/Taunus(DE)**
Erfinder: **Wink, Joachim, Dr.**
**Bieberer Strasse 133**
**D-6050 Offenbach(DE)**
Erfinder: **Hammann, Peter, Dr.**
**Mörikestrasse 6**
**D-6233 Kelkheim(Taunus)(DE)**
Erfinder: **Giani, Carlo, Dr.**
**Hedderichstrasse 69**
**D-6000 Frankfurt am Main 70(DE)**
Erfinder: **Kricke-Helling, Pia, Dr.**
**Welfenweg 1**
**D-3406 Bovenden(DE)**

(54) **Neue Streptenole aus Streptomyceten, ihre Herstellung und ihre Verwendung.**

(57) Aus der Nährlösung von Streptomyceten konnten neue Streptenole mit pharmakologischer Wirkung, insbesondere mit inhibierender Wirkung auf die HMG-CoA-Reduktase, isoliert werden. Die Verbindungen können auch als Synthesebausteine zur Herstellung von Meviloninanaloga eingesetzt werden.

EP 0 385 264 A2

## Neue Streptenole aus Streptomyceten, ihre Herstellung und ihre Verwendung

Streptenole sind hydroxylierte Aliphaten, die in der Natur im wesentlichen von Streptomyceten synthetisiert werden.

Es wurde bereits ein Molekül dieser Substanzklasse [Keller-Schierlein et al. Helvetica Chimica Acta 66, 1253 (1983)] beschrieben, das jedoch gegen Bakterien keine biologische Wirkung besitzt und gegen einzelne Pilze nur eine sehr geringfügige Hemmwirkung zeigt. Die Verbindung wird von Streptomyces fimbriatus synthetisiert. Durch Fermentation des Streptomyceten DSM 4356 wurden nun neben dem bereits bekannten Streptenol neue Verbindungen dieser Gruppe gefunden.

Diese neuen Verbindungen inhibieren die Hydroxymethylglutaryl (HMG)-CoA-Reduktase des Fettstoffwechsels und können als Synthesebausteine insbesondere zur Herstellung von Meviloninanaloga, den wirksamsten HmG-CoA-Reduktase-Inhibitoren, eingesetzt werden.

Die Erfindung betrifft somit:

1. Die Verbindung der allgemeinen Formel I,

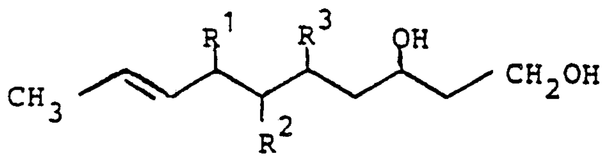

(I)

in der $R^1$ und $R^2$ für Wasserstoff stehen oder zusammen eine Doppelbindung bilden und
$R^3$ für eine Oxogruppe oder eine Hydroxylgruppe steht,
ausgenommen die Verbindung der allgemeinen Formel I, in der $R^1$ und $R^2$ für Wasserstoff stehen und $R^3$ für eine Oxogruppe steht.

2. Ein Verfahren zur Herstellung der unter 1. charakterisierten Verbindung der allgemeinen Formel I, das dadurch gekennzeichnet ist, daß ein Stamm der Gattung Streptomyces in einer Nährlösung kultiviert wird, bis sich die genannte Verbindung in der Nährlösung anhäuft.

3. Die Verwendung der unter 1. charakterisierten Verbindung der allgemeinen Formel I als Inhibitor der HMG-CoA-Reduktase bzw. als Synthesebaustein zur Herstellung von Meviloninanaloga.

Im folgenden wird die Erfindung detailliert beschrieben, insbesondere in ihren bevorzugten Ausführungsformen. Ferner wird die Erfindung in den Ansprüchen definiert.

Die Verbindung der allgemeinen Formel I wird insbesondere von einem Streptomyceten-Stamm hergestellt, der bei der Deutschen Sammlung von Mikroorganismen mit der Nummer DSM 4356 unter den Bedingungen des Budapester Vertrages hinterlegt wurde.

Die Sporenmorphologie von Streptomyces spec. DSM 4356 kann wie folgt charakterisiert werden:
Sporenfarbe: grau
Sporenkette: enge Spiralen
Sporenoberfläche: stachelig

Anstelle des genannten Stammes können auch jeweils dessen Mutanten und Varianten eingesetzt werden, sofern sie die Verbindung der allgemeinen Formel I synthetisieren. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N′-nitro-N-nitroso-guanidin (MNNG) oder 2-Hydroxy-4-methoxybenzophenon (MOB) erzeugt werden.

Als bevorzugte Kohlenstoffquelle für die aerobe Fermentation eignen sich assimilierbare Kohlenhydrate und Zuckeralkohole, wie Glucose, Lactose oder Mannit, sowie kohlenhydrathaltige Naturprodukte, wie Malzextrakt. Als bevorzugte stickstoffhaltige Nährstoffe kommen in Betracht:
Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali- oder Erdalkalimetalle, Eisen, Zink und Mangan als zusätzliche anorganische Salze enthalten.

Die Bildung der Verbindung der allgemeinen Formel I verläuft besondes gut in einer Nährlösung, die Sojamehl und Mannit in Konzentrationen von jeweils 0,5 bis 6 %, bevorzugt 1 bis 4 %, enthält, bezogen auf das Gewicht der gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35° C, vorzugsweise bei etwa 25 bis 30° C, insbesondere bei 28 bis 30° C erfolgen. Man kultiviert den Mikroorganismus unter den genannten Bedingungen bis die stationäre Phase erreicht ist, etwa 60 bis 120 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur, des Mycelvolumens, durch Dünnschichtchromatographie oder Ausprüfen der biologischen Aktivität überwacht werden.

Die Isolierung der Streptenole der allgemeinen Formel I aus dem Kulturmedium erfolgt nach bekannten Methoden unter Berücksichtigung der chemischen, physikalischen und biologischen Eigenschaften der Produkte. Die Streptenole der allgemeinen Formel I liegen im Mycel bzw. in der Kulturbrühe vor. Sie können aus der unfiltrierten Kulturbrühe mit einem mit Wasser nicht oder nur wenig mischbaren organischen Lösemittel, wie Chloroform oder Ethylacetat, extrahiert werden. Da sie sich jedoch nur zu einem geringen Teil im Mycel befinden, ist es vorteilhaft, die Kulturbrühe vom Mycel zu trennen, beispielsweise durch Zentrifugieren oder Filtrieren, vorzugsweise unter Zugabe von Filterhilfsmitteln. Die Verbindung der allgemeinen Formel I kann dann aus dem Überstand bzw. Filtrat isoliert werden, zweckmäßig im leicht sauren bis neutralen pH-Bereich, vorzugsweise bei pH 6 bis 7. Dazu kann man mit Wasser wenig oder nicht mischbare organische Lösemittel verwenden, insbesondere chlorierte Kohlenwasserstoffe, wie Chloroform oder Methylenchlorid oder Ester wie Ethylacetat oder Aceton.

Statt durch Extraktion können die Streptenole auch durch Adsorption an handelsüblichen Adsorberharzen aus der Kulturbrühe isoliert werden. Es hat sich ebenfalls als günstig erwiesen, den genannten Fermenterinhalt zu trocknen, beispielsweise durch Sprühtrocknen oder Gefriertrocknen.

Zur Isolierung der reinen Streptenole können die üblichen Verfahrensschritte Verwendung finden, wie Chromatographie oder Gelfiltration. Besonders bewährt hat sich eine Chromatographie an Kieselgel, wobei als Laufmittel eine Mischung aus Essigester und Hexan in einem Volumenverhältnis von beispielsweise 1:2 Verwendung findet.

Die Streptenole der allgemeinen Formel I sind ölig und gut in Methanol, Aceton, Dimethylsulfoxid, Dioxan und Chloroform löslich, jedoch wenig in Wasser und nicht in Alkanen.

Die erfindungsgemäßen Streptenole inhibieren die HMG-CoA-Reduktase und können damit in vivo die Cholesterinmenge im Organismus reduzieren. Weiterhin können die Verbindungen auch zur Synthese von Meviloninanaloga verwendet werden.

**Beispiele:**

1. **Fermentation des Produzentenstammes DSM 4356**

a) Herstellung einer Sporensuspension des Produzentenstamms:
100 ml Nährlösung (4 g Hefeextrakt, 10 g Malzextrakt, 4 g Glucose, 1 l Leitungswasser, pH-Wert vor dem Sterilisieren 7,3) in einem 500 ml Erlenmeyerkolben werden mit dem Stamm beimpft und 72 Stunden bei 27° C und 120 UpM auf der rotierenden Schüttelmaschine inkubiert. Anschließend werden 20 ml Kulturflüssigkeit in einem 500 ml Erlenmeyerkolben mit dem Nährboden der oben genannten Zusammensetzung, dem 20 g Agar/l zur Verfestigung zugegeben wurde, gleichmäßig verteilt und dekantiert. Die Kulturen werden 10 bis 14 Tage bei 27° C inkubiert. Die nach dieser Zeit entstandenen Sporen eines Kolbens werden mit 500 ml entionisiertem Wasser, das einen Tropfen eines handelsüblichen nichtionischen Tensids enthält, abgeschwemmt, sofort weiterverwendet oder bei -22° C aufbewahrt.

b) Herstellung einer Kultur bzw. Vorkultur eines Produzentenstammes im Erlenmeyerkolben:
Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (pH 7.2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27° C inkubiert. Die maximale Produktion des gewünschten Stoffes ist nach 72 Stunden erreicht. 10 und 100 l Fermenter werden 5 %ig mit einer 48 Stunden alten Submerskultur

aus der gleichen Nährlösung angeimpft.

c) Herstellung der Streptenole:

Ein 10 l Fermenter wird unter folgenden Bedingungen betrieben:

Nährmedium:

2 % Mannit

2 % Sojamehl

pH 7,2

Inkubationszeit: 72 Stunden

Inkubationstemperatur: 30 °C

Rührergeschwindigkeit: 250 UpM

Belüftung: 4 l Luft/Min.

Durch wiederholte Zugabe weniger Tropfen ethanolischer Polyollösung kann die Schaumentwicklung unterdrückt werden. Das Produktionsmaximum wird nach ca. 70 Stunden (pH = 5,3) erreicht. Die Ausbeuten liegen bei ca. 20 mg/l.

2. **Isolierung der Streptenole**

Nach der Fermentation der Produzentenstämme wird die Kulturbrühe unter Zusatz von 2 % Celite als Filterhilfsmittel filtriert. Das Mycel wird mit Ethylacetat extrahiert und die organische Phase eingedampft. Das Kulturfiltrat wird getrocknet und der Rückstand mit Essigester extrahiert. Das Rohprodukt wird an einer Kieselgelsäule (Kieselgel 60, Macherey-Nagel) mit Essigester/Hexan (1:2; v:v) chromatographiert.

3. **Charakterisierung der Streptenole**

a) Charakterisierung der Verbindung:

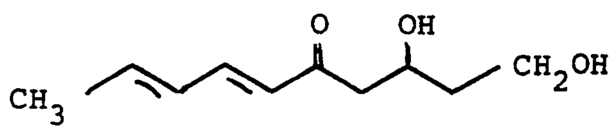

$^{13}$C-NMR ($CDCl_3$, 75 MHz):

$\delta$ = 18,8; 37,9; 46,5; 61,1; 68,0; 127,7; 130,2; 141,5; 144,3; 200,9 ppm.

b) Charakterisierung der Verbindung

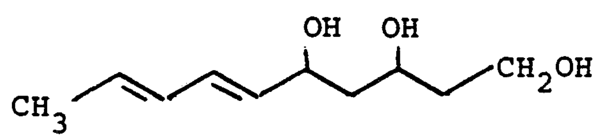

$^{13}$C-NMR ($CDCl_3$, 75 MHz):

$\delta$ = 18,2; 41,1; 45,7; 60,1; 66,6; 69,7; 129,8; 130,9; 132,3; 134,8 ppm.

c) Charakterisierung der Verbindung:

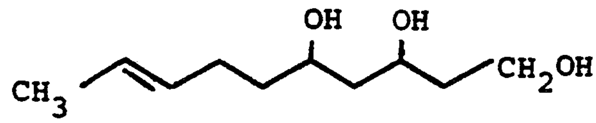

$^{13}$C-NMR ($CDCl_3$, 75 MHz):

$\delta$ = 17,9; 36,0; 37,0; 38,3; 42,6; 61,9; 69,1; 69,8; 125,6; 130,7 ppm.

4. **Hemmung der Cholesterinbiosynthese**

Die biologische Wirksamkeit der Verbindung der Formel I wurde mit Zellkulturen der HEP-G2-Zellinie bestimmt. Dazu wurden Monolayers von HEP-G2-Zellen in lipoproteinfreiem Nährmedium mit entsprechenden Konzentrationen der Prüfsubstanzen eine Stunde vorinkubiert; nach Zugabe des $^{14}C$-markierten Präkursors $^{14}C$-Natriumacetat wird die Inkubation für 3 Stunden fortgesetzt. Danach wird ein Teil der Zellen alkalisch verseift, bei vorheriger Zugabe eines internen Standards von$^{3}H$-Cholesterin. Die Lipide der verseiften Zellen werden mit Chloroform-Methanol extrahiert. Dieses Lipidgemisch wird nach Zusatz von Trägercholesterin präparativ dünnschichtchromatographisch getrennt, die Cholesterinbande nach dem Sichtbarmachen isoliert und die aus dem $^{14}C$-Präkursor gebildete Menge $^{14}C$-Cholesterin szintigraphisch bestimmte. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (lichtmikroskopisch) beurteilt und biochemisch durch Bestimmung der Laktat-Dehydrogenase-Menge im Inkubationsmedium gemessen.

Als Standardpräparat wurde Lovastatin verwendet. Es wurden verschiedene Konzentrationen von Lovastatin eingesetzt und die Konzentration bestimmt, bei der 50 % der Cholesterinsynthese gehemmt wurde ($IC_{50}$-Wert). Der $IC_{50}$-Wert für Lovastatin lag bei $2 \cdot 10^{-8}$ M. Die $IC_{50}$-Werte für Streptenol 3a bis 3c lagen im gleichen Konzentrationsbereich.

## Ansprüche

1. Verbindung der allgemeinen Formel I,

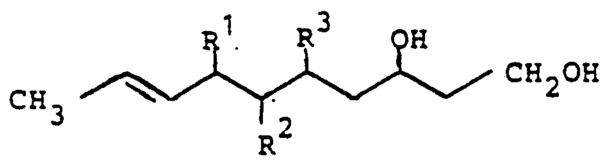

(I)

in der $R^1$ und $R^2$ für Wasserstoff stehen oder zusammen eine Doppelbindung bilden und
$R^3$ für eine Oxogruppe oder eine Hydroxylgruppe steht,
ausgenommen die Verbindung der allgemeinen Formel I, in der $R^1$ und $R^2$ für Wasserstoff stehen und $R^3$ für eine Oxogruppe steht.

2. Verfahren zur Herstellung der Verbindung der allgemeinen Formel I,

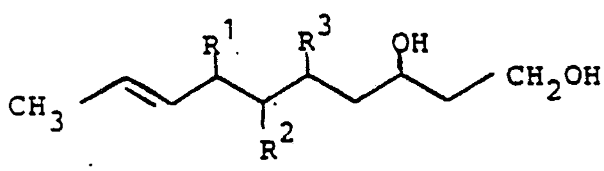

(I)

in der $R^1$ und $R^2$ für Wasserstoff stehen oder zusammen eine Doppelbindung bilden und
$R^3$ für eine Oxogruppe oder eine Hydroxylgruppe steht,
dadurch gekennzeichnet, daß Streptomyces spec. DSM 4356 kultiviert wird bis sich die genannte Verbindung in der Nährlösung anhäuft.

3. Verfahren nach Anspruch 2, dadurch gekennzeichnet, daß die Nährlösung Mannit in Konzentrationen von 0,5 bis 6 % und Sojamehl in Konzentrationen von 0,5 bis 6 %, jeweils bezogen auf das Gewicht der gesamten Nährlösung, enthält.

4. Verfahren nach Anspruch 3, dadurch gekennzeichnet, daß die Nährlösung 2 bis 4 % Mannit und 2 bis 4 % Sojamehl enthält, bezogen auf das Gewicht der Nährlösung.

5. Verfahren nach einem oder mehreren der Ansprüche 2 bis 4, dadurch gekennzeichnet, daß die Kultivierung in einem Temperaturbereich von 18 bis 40 °C erfolgt.

6. Die Verwendung der Verbindung der allgemeinen Formel I,

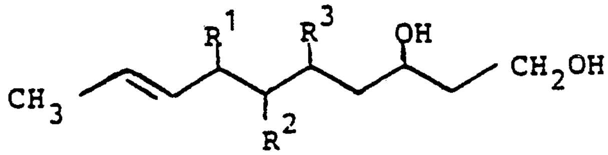

(I)

in der $R^1$ und $R^2$ für Wasserstoff stehen oder zusammen eine Doppelbindung bilden und $R^3$ für eine Oxogruppe oder eine Hydroxylgruppe steht, zur Inhibierung der HMG-CoA-Reduktase.

7. Die Verwendung der Verbindung der allgemeinen Formel I,

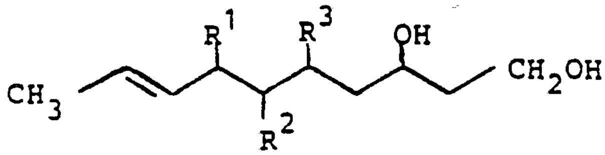

(I)

in der $R^1$ und $R^2$ für Wasserstoff stehen oder zusammen eine Doppelbindung bilden und $R^3$ für eine Oxogruppe oder eine Hydroxylgruppe steht, als Synthesebaustein zur Herstellung von Meviloninanaloga.

8. Streptomyces DSM 4356, sowie dessen Mutanten und Varianten, sofern sie die Verbindung der allgemeinen Formel I,

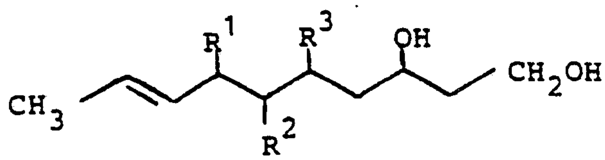

(I)

in der $R^1$ und $R^2$ für Wasserstoff stehen oder zusammen eine Doppelbindung bilden und $R^3$ für eine Oxogruppe oder eine Hydroxylgruppe steht, produzieren.